# EUROPEAN PATENT APPLICATION

(11) **EP 1 611 912 A1**
(43) Date of publication of application: **04.01.2006**
(21) Application number: 05253962.4
(22) Date of filing: 27.06.2005
(51) Int. Cl.: A61M 16/00, A61M 16/10

(54) **Integrated control of ventilator and nebulizer operation**

(30) Priority: 25.06.2004 US 582941; 24.06.2005 US
(71) Applicant: Datex-Ohmeda Inc., Madison, Wisconsin 53707 (US)
(72) Inventor: Tobia, Ronald L., Sun Prairie, Wisconsin 53590 (US); Watson, Anne, Madison, Wisconsin 53717 (US)
(74) Representative: Mosey, Stephen George

(57) **Abstract**

A method of integrating the operations of a ventilator and a nebulizer is provided. In one example, the method includes the steps of (1) providing a control unit in communication with a ventilator and nebulizer, the control unit arranged to obtain a control value based upon one or more ventilatory respiratory control parameters; (2) operating the ventilator; and (3) generating a modification signal from the control unit to automatically modify an operating condition of the nebulizer based upon the control value. In another example, the method includes the steps of (1) providing a control unit in communication with the ventilator and the nebulizer; (2) operating the ventilator and nebulizer, the nebulizer being operated at predetermined dosing periods; and (3) generating a modification signal from the control unit to automatically modify an operating condition of the ventilator based upon the operation of the nebulizer.

## Description

### CROSS-REFERENCE TO RELATED APPLICATION

This application claims the benefit of Provisional Application No. 60/582,941, filed June 25, 2004.

### FIELD OF THE INVENTION

The present invention relates to a method of integrating control of a ventilator with a nebulizer. The invention further provides a method for integrating the active behaviors of a ventilator, nebulizer, and a respiratory monitoring device.

### BACKGROUND OF THE INVENTION

Clinicians commonly utilize a nebulizer to provide aerosolized drug delivery to a patient that is connected to a ventilator. Nebulizers are typically placed in the inspiratory limb of a patient circuit and are used to inject the aerosolized drug directly into the flow stream of the breathing gases for the patient. Prior art nebulizers are typically pneumatic or ultrasonic technology-based devices that are run continuously for a period of time until delivery of discrete doses of the drug has been completed. When used in this fashion, nebulizers introduce aerosolized drug during both the inspiratory and expiratory phases of ventilation, causing a significant portion of the drug dose to bypass the patient and "wrap around" the breathing circuit, exiting through the ventilator's exhalation valve. Essentially, this drug is wasted, as it is not delivered to the patient.

Recently, a new type of nebulizer has been introduced that utilizes "micro-pump" technology. The micro-pump forces liquid drug through a fine sieve (e.g. 3 microns) to produce the aerosol drug delivery. An advantageous characteristic of this technology is that the onset and suspension of the nebulization operation can be conducted very quickly, thus making this nebulizer ideal for controlling drug delivery during specific periods of a breath phase. Operation of nebulizers in this manner has been discussed in prior art literature, and in particular, Piper et al U.S. Patent No. 5,479,920 and Raabe et al U.S. Patent No. 5,322,057.

While the prior art nebulizer systems describe methods for operating a nebulizer intermittently during specific breath phases, optimal delivery of aerosolized drug to a patient depends on a number of parameters that are not directly related to the breath phase information. One example of such a parameter is the drug type itself, as it is desirable to deposit certain drugs (e.g. vasodilators) deep into the patient's lungs and other drugs (e.g. bronchodilators) only into the patient's upper airway. Other parameters related to nebulizer optimization include the location of the nebulizer in the patient circuit (e.g. at the patient wye, upstream in the inspiratory limb), breathing circuit type and volume, the ventilator's inspiratory flow rates, the breath delivery type (e.g. pressure or volume, spontaneous or mechanical), and the ventilator's bias (i.e. base or "wrap-around") flow rate. Prior art nebulizer systems, even those that operate the nebulizer intermittently during a breath, do not respond to these parameters and therefore produce less than optimal drug delivery therapy.

Prior art nebulizer systems are also not integrated with systems providing respiratory mechanics monitoring. Thus, clinical information regarding the effectiveness of nebulized drug therapy must be solicited independent of the nebulizer's operation. The prior art ventilation and monitoring devices thus require individual adjustments by the user to affect a desired therapy or simple physiologic measurement behavior. This can require time-consuming and tedious manual operations and therefore undesirably reduces system efficiency.

It is desirable to link the nebulizer's function with respiratory mechanics monitoring to increase the efficiency and efficacy of the monitoring assessment of drug therapy effectiveness. Further, use of this integration would allow the nebulized drug therapy to be controlled by the results of the monitoring information. In this manner, the common occurrence of delivering nebulized drug for periods well in excess of its full effectiveness being realized can be eliminated.

As such, it is desirable to provide a method for integrating ventilator and nebulizer operations. It is desirable to provide integrated ventilator control of nebulizer operation such that the amount and time-of-delivery of the aerosolized drug is optimized. It is further desirable to provide such integrated ventilator control of nebulizer operation such that the nebulizer is automatically operated according to at least one of a series of the parameters discussed above. Also, it is desirable to provide such integrated ventilator control of nebulizer operation with integrated respiratory monitoring capabilities such that the clinician is able to efficiently assess the effectiveness of nebulized drug therapy. Finally, it is desirable to utilize this automatic drug effectiveness assessment to automatically control nebulized drug delivery, until a desired effect has been obtained.

### SUMMARY OF THE INVENTION

The present invention provides such a method for integrating the active behaviors of a ventilator and a nebulizer. The invention further provides such a method for integrating the active behaviors of a ventilator, nebulizer, and a respiratory monitoring device.

In a preferred example, the method of integrating the operations of a ventilator and a nebulizer includes the steps of (1) providing a control unit in communication with the ventilator and nebulizer, the control unit arranged to obtain a control value based upon one or more ventilatory respiratory control parameters; (2) operating the ventilator; and (3) generating a modification signal from the control unit to automatically modify an operating condition of the nebulizer based upon the control value.

In a preferred embodiment, an integrated ventilator and a piezo-electric micro-pump nebulizer provides the fundamental ability to control the nebulizer (for example, to turn the nebulizer on or off via an electrical control signal) during various phases of the patient's breath. This fundamental ability provides optimization of aerosolized drug delivery by allowing for automatic adjustment of the synchronization skew and/or period of nebulization within the breath cycle in response to at least one of several parameters obtained by the integrated ventilator and nebulizer. The parameters may include: drug type; information obtained during checkout of the ventilator; patient circuit type; patient circuit volume; patient circuit compliance; patient circuit length; position of nebulizer in patient circuit; inspiratory flow rate of the ventilator; peak inspiratory flow rate of the ventilator; inspiratory volume of the ventilator; bias flow rate of the ventilator; and/or breath control type of the ventilator, e.g. pressure or volume. Thus the integrated method of the present invention allows the intermittent periods of drug delivery from the nebulizer to be optimally matched with the gas delivery to the patient to achieve the most effective delivery for a given drug regimen.

In another preferred example, the method of integrating operations of a ventilator and a nebulizer to conduct respiration therapy includes the steps of (1) providing a control unit in communication with the ventilator and the nebulizer; (2) operating the ventilator and nebulizer, the nebulizer being operated at predetermined dosing periods; and (3) generating a modification signal from the control unit to automatically modify an operating condition of the ventilator based upon the operation of the nebulizer.

Integration of the ventilator and the nebulizer further allows for automatic modification of ventilation delivery in response to "dosing periods" (i.e. periods when the nebulizer is being intermittently operated to deliver a drug therapy) of a nebulizer, and more specifically a piezo-electric pump, such that: (1) bias flow is increased or decreased during periods of nebulizer dosing; (2) inspired breath profiles (e.g. pressure or volume) are modified during periods of nebulizer dosing; (3) inspired flow profiles are modified during periods of nebulizer dosing; (4) inspiratory time (including inspiratory pause) is modified during periods of nebulizer dosing; (5) expiratory time (including expiratory pause) is modified during periods of nebulizer dosing; and/or (6) breath rate is modified during periods of nebulizer dosing.

In another preferred example, a method of integrating the operations of a ventilator, a nebulizer, and a respiratory monitoring device is provided. The method includes the steps of (1) providing a control unit in communication with the ventilator, the nebulizer, and the respiratory monitoring device, the respiratory monitoring device arranged to obtain a monitoring value based upon one or more respiratory monitoring parameters; (2) operating the ventilator and nebulizer; and (3) generating a modification signal from the control unit to automatically modify an operating condition of the nebulizer based upon the control value.

Integration of the ventilator and the nebulizer with monitoring capabilities allows for the initiation, completion or intermediate point of the nebulizer dose delivery to trigger automatic monitoring functions. For example, one embodiment of the present invention comprises the integration of ventilation delivery, nebulized drug delivery and respiratory monitoring, namely respiratory mechanics monitoring and/or respiratory gas monitoring, where respiratory mechanics monitoring includes one or more of the following: (1) functional residual capacity measurements; (2) lung pressure and airway resistance measurement; (3) compliance measurement; (4) resistance measurement; (5) pressure-volume loop; (6) flow-volume loop; and (7) pressure-flow loop and respiratory gas monitoring includes one of more of the following (1) end tidal CO2; (2) CO2 production,; (3) end tidal 02; and (4) 02 consumption. Using this embodiment, the monitoring information so obtained can be used to automatically control the start and/or termination of the dose period(s) provided by the nebulizer.

### BRIEF DESCRIPTION OF THE DRAWINGS

Preferred embodiments of the invention are described herein below with reference to the attached drawing figures, wherein:

Figure 1 is a schematic illustration of a prior art configuration for a ventilator, nebulizer and gas monitoring device.

Figure 2 is a schematic illustration of the integration of control for the ventilator, drug delivery device and gas measurement device.

Figure 3 is a graph depicting a typical breathing cycle pressure waveform.

Figure 4 is a schematic depicting the steps of a method whereby the a function of a nebulizer is changed automatically based on one or more control parameters from the ventilator and/or breath phase information.

### DETAILED DESCRIPTION OF THE INVENTION

In the preferred embodiments of the present invention described in detail below, a method for integrating the active behaviors of a ventilator and a nebulizer is provided. It should be understood that the drawings and specification are to be considered an exemplification of the principles of the invention. For example, although the concepts of the invention are described with reference to the Engstrom Carestation®, which has intensive care therapy applications, the present invention is also applicable in many other patient care settings, such as in anesthesia settings or emergency room settings. For example, a ventilator designed to provide Heliox as breathing gas in combination with an integrated nebulizer system would have application in the treatment of asthmatic patients in an emergency room setting.

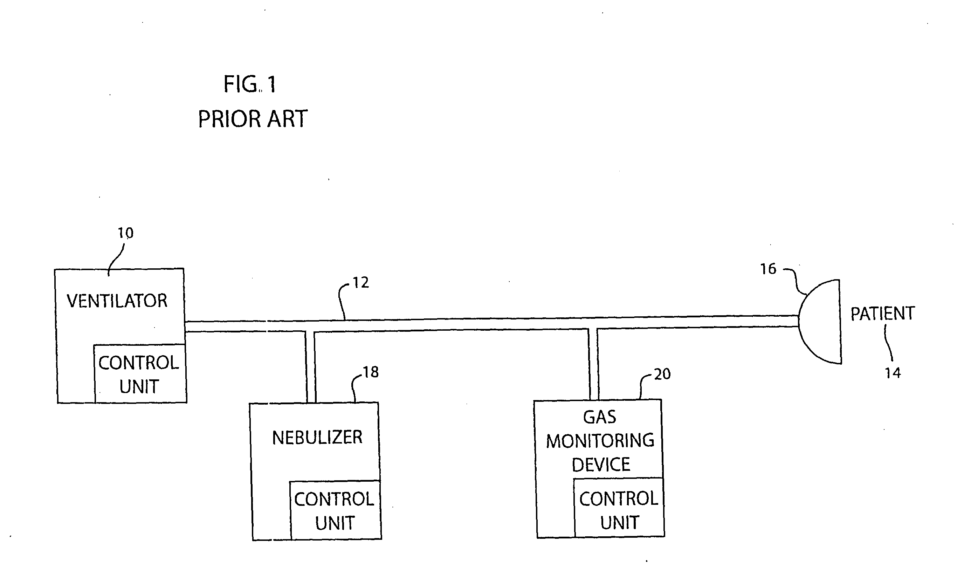

Referring to Fig. 1, a typical configuration of a ventilation system for providing a flow of ventilation gas to a patient is shown. A ventilator 10 provides a flow of ventilation gas through a patient conduit 12 to the patient 14. In the embodiment illustrated in Fig. 1, the patient receives the flow of ventilation gas through a gas mask 16, although various other types of delivery connections are contemplated as being within the scope of the present invention.

The ventilator and other associated devices can be operated to carry out various different respiratory therapy procedures. As shown in Fig. 1, a nebulizer 18 is provided to medicate a patient on the ventilator. The nebulizer 18 introduces aerosolized medication periodically as prescribed into the breathable gas flowing through the inspiratory patient conduit 12 of the patient circuit and ultimately to the patient's airway and lungs. Additionally, the patient conduit 12 can be coupled to a respiratory gas monitoring device 20 that operates to monitor the contents and quality of the ventilator flow to the patient 14.

As can be understood in Fig. 1, the ventilator 10, nebulizer 18 and respiratory gas monitoring device 20 do not communicate with each other. During operation of the nebulizer 18, the operation of the gas monitoring device 20 must be manually suspended while the nebulizer 18 introduces the aerosolized medication in to the patient conduit 12. In addition, known nebulizer technology 18 includes pneumatic or ultrasonic technology based devices that are run continuously for a period of time until delivery of discrete doses of the drug has been completed. When used in this fashion, nebulizers introduce aerosolized drug during both inspiratory and expiratory phases of ventilation, causing a significant portion of the drug dose to bypass the patient and "wrap around" the breathing circuit exiting through the ventilators exhalation valve. Essentially, this drug is wasted as it is not delivered to the patient.

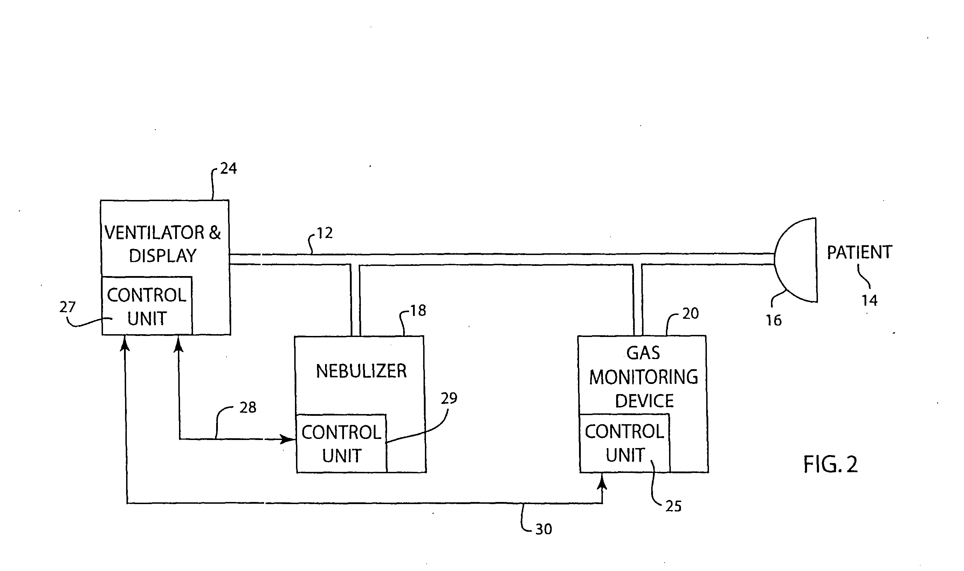

Referring now to Fig. 2, there shown is a preferred configuration for a system for use with the present invention. As illustrated in Fig. 2, the ventilator and integrated display 24 provide a ventilation gas flow through the patient conduit 12 to the patient 14. Likewise, the nebulizer 18 and a monitoring device such as a respiratory gas monitoring device 20 are each in communication with the patient conduit 12. Thus, the nebulizer 18 is able to introduce an aerosolized medication into the flow of gas within the patient conduit 12, while the gas monitoring device 20 is able to take measurements of the ventilator gas flow as desired.

The nebulizer 18 preferably contains micro-pump technology, which, as described above, forces liquid drug through a fine sieve to produce aerosolized drug for delivery to the patient's airway. An example of this technology is a nebulizer marketed under the trademark Aeroneb Pro®.

In the embodiment illustrated in Fig. 2, the control unit 27 of the ventilator and display 24 is in communication with a control unit 29 of the nebulizer 18 over a communication link 28. Likewise, the control unit 27 of the ventilator and display 24 is in communication with control unit 25 of the respiratory gas monitoring device 20 over a second communication link 30. In this manner, the control unit 27 of the ventilator and display 24 can communicate in a bi-directional manner with both the nebulizer 18 and the respiratory gas monitoring device 20.

It is contemplated that the communication links 28, 30, could be replaced by any method of communicating over a point to point network. As an example, electrical and RF communication methodologies are contemplated as being within the scope of the present invention.

An example of a device that electro-mechanically combines a ventilator and a nebulizer is the Engström Carestation® marketed by GE Health Care.

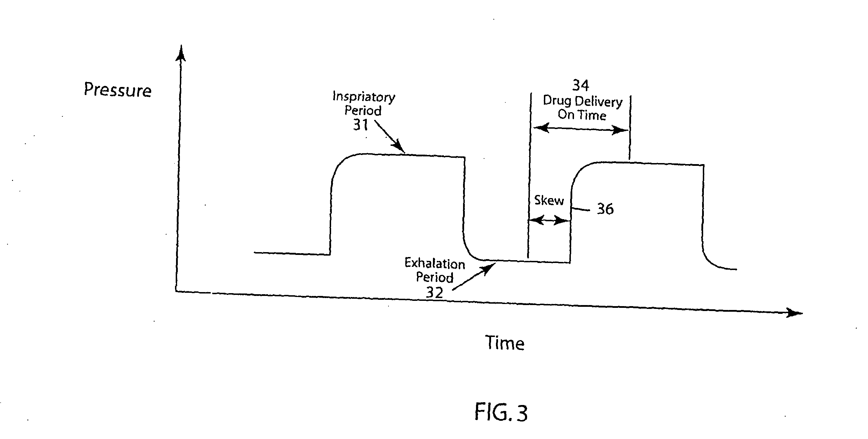

Referring briefly to Fig. 3, a typical breathing cycle pressure waveform is diagrammed. A period of high pressure comprises the inspiration 31 and a period of lower pressure comprises the exhalation period 32. During dosing periods, intermittent nebulized drug delivery occurs synchronously with the breath cycle as defined by the drug delivery "on time" 34 and the synchronization "skew" 36.

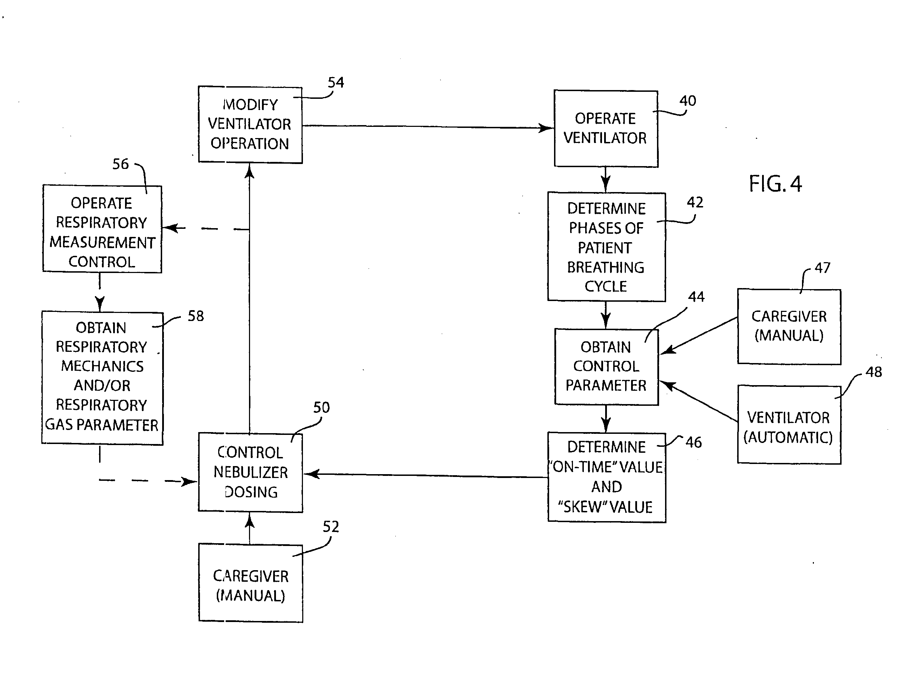

Referring now to Fig. 4, electro-mechanical integration of the nebulizer 18 with the ventilator 27 provides the numerous advantages described above, including the ability to automatically control the delivery of drug to a patient both up until, and after a desired effect has been obtained. At step 40, ventilatory support is provided to the patient by the ventilator 24. During the ventilatory support, the phases of the patient breathing cycle are determined at step 42.

At step 44, a reference value or control parameter is obtained by the ventilator 24. The control parameter may either be entered into the ventilator 24 by a clinician at step 47, or may be automatically determined by the ventilator 24 at step 48. Preferably, the control parameter comprises any one of a variety of parameters that are used to determine the optimal delivery "on time" 34 and "synchronization skew" 36 for the nebulized drug therapy being delivered to the patient. Examples of such a parameter include (1) drug type; (2) information obtained during checkout of the ventilator; (3) patient circuit type; (4) patient circuit volume; (5) patient circuit compliance; (6) patient circuit length; (7) position of the nebulizer in the patient circuit; (8) inspiratory flow rate of the ventilator; (9) peak inspiratory flow rate of the ventilator; (10) inspiratory volume of the ventilator; (11) bias flow rate of the ventilator; and/or (12) breath control type of the ventilator, e.g. pressure or volume.

At step 46, the ventilator automatically determines the optimal delivery "on time" 34 and "synchronization skew" 36 based upon one or more of the control parameters described above and then automatically controls the function of the nebulizer 18 at step 50. Such automatic control allows the system to provide a certain amount of the aerosolized drug in the most efficient manner possible. The clinician can also start or stop the dosing period manually at step 52.

As a further or alternative step 54, the ventilation delivery can be modified to facilitate optimized nebulization during dosing periods controlled in step 50. For example, integration of the ventilator 24 and the nebulizer 18 further allows for automatic modification of ventilation delivery in response to operation of the nebulizer 18 such that: (1) bias flow is increased or decreased during periods of nebulizer dosing; (2) inspired breath profiles (e.g. pressure or volume) are modified during periods of nebulizer dosing; (3) inspired flow profiles are modified during periods of nebulizer dosing; (4) inspiratory time (including inspiratory pause) is modified during periods of nebulizer dosing; (5) expiratory time (including expiratory pause) is modified during periods of nebulizer dosing; and/or (6) breath rate is modified during periods of nebulizer dosing.

It is further recognized by the present application that in one system, the nebulizer, ventilator, and patient monitor may all be fully integrated. Such an arrangement has been reduced to practice in the above-reference Engström Carestation® and has been found to greatly enhance the efficiency of the automatic control of patient therapy. For example, as shown in Fig. 4, respiratory measurement is conducted by the gas monitoring device 20 at step 56. At step 58, a respiratory mechanics and/or respiratory gas measurement parameter is obtained.

Respiratory mechanics monitoring may include one or more of the following: (1) residual capacity measurements; (2) lung pressure and airway resistance measurement; (3) compliance measurement; (4) resistance measurement; (5) pressure-volume loop; (6) flow-volume loop; and (7) pressure-flow loop. Respiratory gas monitoring may include one or more of the following: (1) end tidal CO2; (2) CO2 production; (3) end tidal 02; and (4) 02 consumption.

Steps 56 and 58 can be initiated by the automatic control of the nebulizer dosing period at step 60. Using the respiratory mechanics and/or respiratory gas measurement parameter obtained at step 58, the operation of the nebulizer and ventilator can be adjusted to optimize ventilatory therapy.

There are many alternative conceivable methods for integrating the ventilator 24 and the nebulizer 18 and the respiratory monitor 20 such that the above desired efficiency/accuracy is obtained. For example, the ventilator 24 can be integrated with the nebulizer 18 and the monitor 20 wherein:
- Activation of a nebulizer dose generates a monitoring sample of respiratory mechanics and/or respiratory gas parameters.
- Completion of a nebulizer dose generates a monitoring sample of respiratory mechanics and/or respiratory gas parameters.
- A monitoring sample of respiratory mechanics and/or respiratory gas parameters is obtained at a preset time relative to nebulization doses. This timing could continue to reoccur. For example the integrated ventilator would sample respiratory mechanics parameters immediately before and 15 minutes after nebulizer doses, while the nebulizer doses themselves are repeated automatically every 2 hours
- Automatic termination or initiation of nebulized drug dosing step 22 in reaction to information provided by the respiratory mechanics and/or respiratory gas monitoring information obtained at step 28. For example, when airway resistance measurements drop an amount specified by the clinician, the integrated ventilation delivery and nebulizer device will terminate the nebulized drug delivery of a bronchodilator. (In contrast, current practice provides for the delivery of a specific amount of drug regardless of effect. Often extra drug is delivered even after its full effectiveness has been realized. This "closed-loop" behavior would allow the clinician to automatically limit the delivery of drug once the desired effect has been obtained.)
While this invention is susceptible to embodiments in many different forms, the drawings and specification describe in detail preferred embodiments of the invention. They are not intended to limit the broad aspects of the invention to the embodiment illustrated.

## Claims

1. A method of integrating the operations of a ventilator and a nebulizer, the method comprising the steps of:
providing a control unit in communication with the ventilator and the nebulizer, the control unit arranged to obtain a control value based upon one or more respiratory control parameters;
operating the ventilator; and
generating a modification signal from the control unit to automatically modify an operating condition of the nebulizer based upon the control value.

2. The method of claim 1, wherein the modification signal automatically adjusts a synchronization skew within a breath cycle.

3. The method of claim 1, wherein the modification signal automatically adjusts a period of nebulization within a breath cycle.

4. The method of claim 1, wherein the one or more control parameters comprise a type of drug dispensed by the nebulizer.

5. The method of claim 1, wherein the one or more control parameters comprise the location of the nebulizer in a breathing circuit associated with the ventilator.

6. The method of claim 1, wherein the one or more control parameters comprise a physical quality of a breathing circuit associated with the ventilator.

7. The method of claim 1, wherein the one or more control parameters comprise an inspiratory flow rate associated with the ventilator.

8. The method of claim 1, wherein the one or more control parameters comprise a bias flow rate associated with the ventilator.

9. The method of claim 1, wherein the one or more control parameters comprise a breath delivery type associated with the ventilator.

10. A method of integrating the operations of a ventilator and a nebulizer, the method comprising the steps of:
providing a control unit in communication with the ventilator and the nebulizer;
operating the ventilator and nebulizer, the nebulizer being operated at selected dosing periods; and
generating a modification signal from the control unit to automatically modify an operating condition of the ventilator based upon the operation of the nebulizer.

11. The method of claim 10, wherein the operating condition of the ventilator affects a bias flow value.

12. The method of claim 10, wherein the operating condition of the ventilator affects an inspired breath profile.

13. The method of claim 10, wherein the operating condition of the ventilator affects an inspiratory time provided by the ventilator.

14. The method of claim 10, wherein the operating condition of the ventilator affects an expiratory time provided by the ventilator.

15. The method of claim 10, wherein the operating condition of the ventilator affects a breath rate provided by the ventilator.

16. A method of integrating the operations of a ventilator, a nebulizer, and a respiratory monitoring device, the method comprising the steps of:
providing a control unit in communication with the ventilator, nebulizer, and respiratory monitoring device, the respiratory monitoring device arranged to obtain a monitoring value based upon one or more respiratory monitoring parameters;
operating the ventilator and nebulizer; and
generating a modification signal from the control unit to automatically modify an operating condition of the nebulizer based upon the control value.

17. The method of claim 17, wherein the monitoring parameter is based on respiratory mechanics monitoring.

18. The method of claim 18 wherein the respiratory mechanics monitoring includes at least one of functional residual capacity measurement, lung pressure and airway resistance measurement, compliance measurement, resistance measurement, pressure-volume loop, flow-volume loop, and pressure-flow loop.

19. The method of claim 17 wherein the monitoring parameter is based on respiratory gas monitoring.

20. The method of claim 19 wherein the respiratory gas monitoring includes at least one of tidal CO₂, CO₂ production, end title O₂ and O₂ consumption.
